(19) Europäisches Patentamt
European Patent Office
Office européen des brevets

(11) Publication number: **0 487 104 A1**

(12) **EUROPEAN PATENT APPLICATION**

(21) Application number: **91119961.0**

(51) Int. Cl.[5]: **C12Q 1/68**

(22) Date of filing: **22.11.91**

(30) Priority: **22.11.90 JP 319169/90**

(43) Date of publication of application:
**27.05.92 Bulletin 92/22**

(84) Designated Contracting States:
**CH DE FR GB IT LI NL**

(71) Applicant: **SUMITOMO ELECTRIC INDUSTRIES, LTD**
**5-33, Kitahama 4-chome, Chuo-ku**
**Osaka-shi, Osaka-fu(JP)**

(72) Inventor: **Kishimoto, Toshihiko, c/o Osaka Works**
**Sumimoto Electric Ind. Ltd., 1-3, Shimaya 1-chome**
**Konohana-ku Osaka(JP)**
Inventor: **Niwa, Shin-ichiro, c/o Osaka Works**
**Sumimoto Electric Ind. Ltd., 1-3, Shimaya 1-chome**
**Konohana-ku Osaka(JP)**
Inventor: **Nakabayashi, Makoto, c/o Osaka Works**
**Sumimoto Electric Ind. Ltd., 1-3, Shimaya 1-chome**
**Konohana-ku, Osaka(JP)**

(74) Representative: **Hansen, Bernd, Dr.rer.nat. et al**
**Hoffmann, Eitle & Partner Patentanwälte**
**Arabellastrasse 4 Postfach 81 04 20**
**W-8000 München 81(DE)**

(54) **Method of immobilizing single-stranded DNA on carrier at terminal.**

(57) A method of immobilizing a single-stranded DNA on a carrier at the terminal, which comprises binding a double-stranded DNA comprising the single-stranded DNA and its complementary strand to the carrier only at the terminal of the single-stranded DNA and subsequently detaching the complementary strand from the single-stranded DNA by means of denaturing treatment, provided that the single-stranded DNA contains one or more extra nucleotide molecule(s) at the terminal when compared with the complementary strand or the terminal nucleotide molecule of the single-stranded DNA has been chemically modified so that the single-stranded DNA can bind to the carrier via the extra nucleotide molecule(s) or the chemically-modified terminal nucleotide molecule.



Rank Xerox (UK) Business Services

The present invention relates to the field of molecular biology, and more particularly, it relates to a method of immobilizing a single-stranded DNA on a carrier at the terminal.

Single-stranded DNAs immobilized on a carrier have long been used in biotechnology. For example, an oligo-dT DNA immobilized in a solid particle, such as oligo-dT cellulose, is used for isolating mRNA from a mixture of RNAs of every sort. A single-stranded DNA nonspecifically immobilized on a membrane is used in experiments involving hybridization, such as Southern Hybridization.

Such immobilized single-stranded DNAs which have conventionally been used are prepared by binding a single-stranded DNA at the terminal nucleotide molecule of the strand to a carrier via an amino or hydroxy group present on the terminal molecule or a suitable functional group introduced into the molecule. However, such conventional method has a drawback that it is impossible to bind the single-stranded DNA to the carrier only at its terminal molecule by the use of the conventional method because an existing amino or hydroxy group, or other functional group artificially introduced, on the nucleotide molecules other than terminal also participates in the binding with the carrier. The immobilized DNA thus obtained by conventional methods is the one in which the DNA molecules are bonded to the carrier at various sites of the strand. As will be recognized by those skilled in the art, such immobilized DNA which contains DNA strands bonded to the carrier at their non-terminal sites cannot afford to provide sufficient experimental results in any aspects.

The inventors of the present invention have paid attention to the fact that the reactivity of an amino or hydroxy group contained in the nucleotide molecules of a single-stranded DNA can be blocked by annealing the single-stranded DNA with its complementary strand, and they developed the following unique strategies, utilizing the complementarity of the DNA strand, to bind a single-stranded DNA to a carrier only at its terminal. Thus, the terminal nucleotide molecule of a single-stranded DNA is chemically modified so that the DNA can bind to a carrier through the modified terminal molecule. The DNA is then subjected to annealing with its complementary strand and allowed to react and bind with the carrier. Alternatively, a double-stranded DNA in which one of the strands has one or more extra nucleotide molecules at the terminal may be allowed to bind to the carrier via the extra molecules with or without chemical modification of the extra molecules. The double-stranded DNA immobilized on the carrier is subsequently subjected to denaturing treatment, thereby the complementary strand is detached from the single-stranded DNA immobilized on the carrier because the complementary strand is not covalently bonded to the carrier. Accordingly, removal of the detached complementary strand gives a single-stranded DNA immobilized on the carrier exclusively at the terminal molecule. The present invention is based on the above findings.

Thus, one aspect of the present invention is to provide a method of immobilizing a single-stranded DNA on a carrier at the terminal, which comprises binding a double-stranded DNA comprising the single-stranded DNA and its complementary strand to the carrier only at the terminal of the single-stranded DNA and subsequently detaching the complementary strand from the single-stranded DNA by means of denaturing treatment, provided that the single-stranded DNA contains one or more extra nucleotide molecule(s) at the terminal when compared with the complementary strand or the terminal nucleotide molecule of the single-stranded DNA has been chemically modified so that the single-stranded DNA can bind to the carrier via the extra nucleotide molecule(s) or the chemically-modified terminal nucleotide molecule. Another aspect of the invention is to provide a single-stranded DNA immobilized at the terminal on a carrier, which is obtained by the above-mentioned method.

The invention is described in more detail below.

Chemical modification of the terminal nucleotide of a single-stranded DNA

1. A single-stranded DNA, into which a functional group (e.g., $-NH_2$, -COOH, etc.) capable of reacting and binding with a carrier has been introduced at the terminal nucleotide, is first prepared. A single-stranded DNA can be prepared by the use of a commercially available DNA synthesizer, for instance, Model 391 PCR-MATE EP (supplied from Applied Biosystems Inc. (ABI)) in conventional manner. Introduction of the functional group into the terminal nucleotide may be achieved using one of the following methods.

i) Hexylamino group can be introduced at the terminal nucleotide using Amino Link 2 (supplied from ABI) and DNA synthesizer according to the following reaction schema (See Applied Biosystems Inc. User Bulletin, No. 49, August 1988).

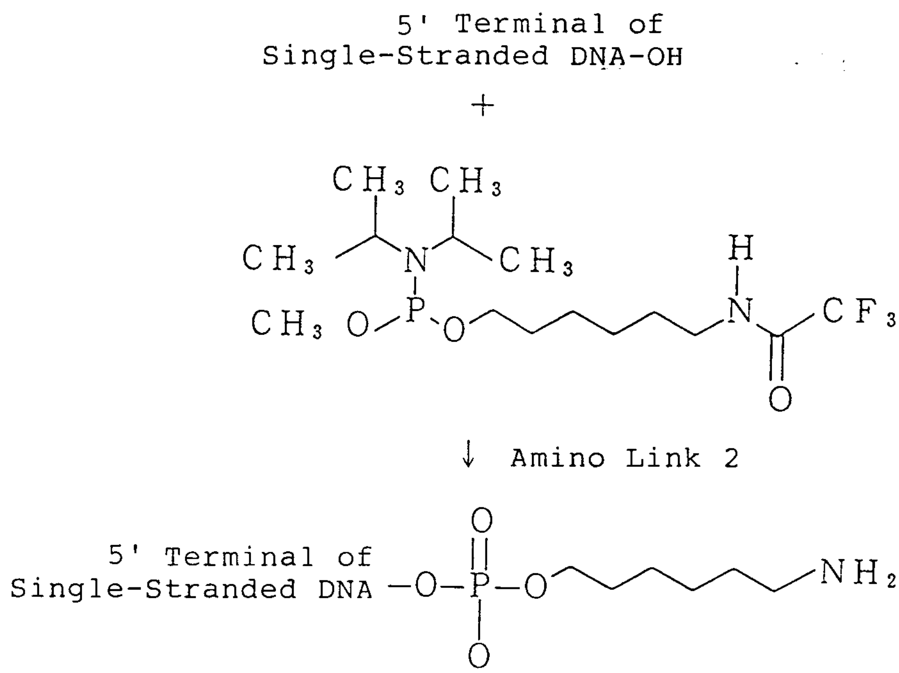


ii) A linker of the following formula:

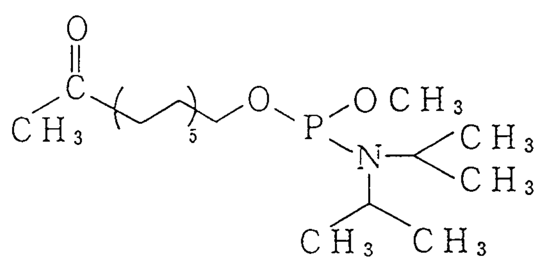

can be introduced into DNA terminal by the use of DNA synthesizer, for instance, Model 391 PCR-MATE EP. The terminal of the linker can be changed to a functional group such as carboxy or aldehyde group by suitable means. The aldehyde group on the linker can be further reacted with biotin hydrazide, thereby providing, at the end of the linker, biotin moiety which can form a complex with avidin (See Jonathan N. Kremsky, Nucleic Acid Research, Vol. 15, p2891-, 1987).

iii) One to tens nucleotide molecules having an amino group are added to the terminal of a single-stranded DNA using DNA synthesizer.

iv) A nucleotide having a functional group suitable for binding to a carrier or a functional derivative thereof is introduced into DNA terminal by the use of terminal transferase (See Deug, G. and Wu, R., Method in Enzymology, Vol. 100, p96-116, 1983).

2. A second DNA strand complementary to the single-stranded DNA obtained above is prepared using a DNA synthesizer, and both strands are subjected to annealing.

3. The double-stranded DNA obtained above is mixed and reacted with a carrier so that the DNA is bound to the carrier through the functional group on the single-stranded DNA.

Carriers on which the single-stranded DNA is immobilized may be in the form of membrane or particles, and specific examples of the carrier are natural or synthetic organo-polymeric membrane or particles such as nylon, nitrocellulose, polytetrafluoroethylene, or polyethylene, inorganic polymeric membrane or particles such as graphite, porous glass, or silica, metal membrane or particles such as aluminum or apatite, ceramic membrane or particles such as alumina or silicon nitride, and crystal sodium chloride.

The above-noted materials can also be used after physical or chemical treatment on their surface. For instance, the organic-polymer may be subjected to chemical treatment such as oxidation, reduction, or hydrolysis, physical treatment such as plasma irradiation. The inorganic polymer, metal, and ceramic

may be subjected to ion-plating or chemical treatment such as oxidation, reduction, or hydrolysis, physical treatment such as plasma irradiation. Specific examples of the surface treatment are treatment of polymethylmethacrylate with 4N NaOH for several hours to generate a carboxyl group on the surface, ultraviolet irratiation on polystyrene film to generate a peroxide on the surface, which is then treated with an acid to form a hydroxy group, treatment of Teflon sheet with sodium naphthalenide and subsequently with hydrogen peroxide to introduce a hydroxy group (Ind. Eng. Chem. Res., Vol. 28, No. 7, 1989), and glow discharge on polyurethane film under ammonia atmosphere to generate an amino group on the surface, chemical treatment of silica with epoxy silane and after that, the expoxy residue introduced is cleaved with an acid to generate a diol group on the surface.

Binding between DNA and a carrier may be conducted in various ways depending on the functional groups present on the terminal nucleotide and the carrier. Several examples are illustrated below.

i) A hydroxy group, preferably diol group, on a DNA or carrier is activated and reacted with an amino group or thiol group e.g. on a carrier or DNA. The activation of a hydroxy group may be conducted using trifluoloethane sulfonyl chloride (K. Nillson and K. Mosbach, Biochem. Biophys. Res. Commun., Vol. 102, 449, 1981), CNBr (R. Axen et al., Nature, Vol. 214, 1302, 1967), trichlorotriazine (T. H. Finlay et al., Anal. Biochem., Vol. 87, 77, 1978), epichlorohydrin (I. Matsumoto et al., J. Biochem., Vol. 85, 1091, 1979), bisoxirane (L. Sundberg and J. Porath, J. Chromatogr., Vol. 90, 87, 1974), divinylsulfonic acid (J. Porath, Meth. Enzymol., Vol. 34, 27, 1974), benzoquinone (J. Brandt et al., Biochem. Biophys. Acta, Vol. 386, 196, 1975), or carbonyldiimidazole (G. S. Bethell et al., J. Biol. Chem., Vol. 254, 2572, 1979).

ii) A carboxyl group on a DNA or carrier is activated and reacted with an amino group on a carrier or DNA. The activation of the carboxyl group may be conducted using a carbodiimide such as water-soluble carbodiimide (A. Tengblad, Biochem. J., Vol. 199, 297, 1981; M. Funabashi et al., Anal. Biochem., Vol. 126, 414, 1982) or 2-ethoxy-1-ethoxycarbonyl-1,2,-dihydroquinoline (EEDQ) (G. Saccomani et al., J. Biochem., Vol. 256, 12405, 1981; B. Belleau and G. Malek, J. Am. Chem. Soc., Vol. 90, 1651, 1986).

iii) DNA of interest is ligated, using DNA ligase, to a DNA non-specifically immobilized on a carrier by conventional manner.

iv) A hydrazide group on DNA and an aldehyde group on a carrier, or vice versa, are reacted each other to form a hydrazone bond. Reduction of the hydrazone makes a covalent bond between the DNA and carrier (Jonathan N. Kremsky, do.). A carboxyl group can be used in place of an aldehyde group, using carbodiimide as a condensing agent.

v) Biotin moiety introduced into DNA is allowed to bind to avidin moiety introduced into a carrier, or vice versa (Jonathan N. Kremsky, do.). Other affinity groups can also be used.

vi) Thiol groups on DNA and carrier are reacted each other to make a bond (K. Brocklehurst et al., Biochem. J., Vol. 133, 573, 1973).

vii) Amino groups on DNA and carrier are reacted each other according to Bromoacetamide method (P. Cuatrecasas, J. Biol. Chem., Vol. 245, 3059, 1970).

4. A double-stranded DNA immobilized on a carrier according to any one of the methods described above is heated at 40 °C or higher, or added with an alkali, in a suitable salt solution such as 2.4M aqueous tetraethylammonium chloride, appropriately diluted 10×SSC (1.5M NaCl, 0.15M sodium citrate, pH7.0), or 0.1-2M NaCl. The mixture is centrifuged to obtain a solid phase comprising a single-stranded DNA immobilized on the carrier.

Use of double-stranded DNA as a starting material with or without modification

As previously stated, a double-stranded DNA which contains one or more extra nucleotide molecules on one of the strands can be bonded to a carrier at the terminal nucleotides with or without chemical modification thereof. Accordingly, the double-stranded DNA immobilized on a carrier can be subjected to the above-noted steps 3 and 4 to obtain a single-stranded DNA immobilized on a carrier. Such double-stranded DNA containing extra nucleotide molecules on one strand may be prepared in the following manner.

i) As previously stated (Chemical Modification, 1-iv)), one or more nucleotides or its derivatives suitable for binding with a carrier can be introduced into only one strand at the terminal by the use of terminal transferase.

ii) Double-stranded DNA is digested with an appropriate restriction enzyme so as to provide one strand moiety at the terminal.

iii) A double-stranded DNA having a functional group at the terminal is ligated to another double-stranded

DNA. For this purpose, a double-stranded DNA is first digested with two different restriction enzymes so that the DNA may have different restriction ends. One of the restriction end is allowed to bind to a DNA carrying a functional group and capable of specifically reacting with said restriction end, using DNA ligase. It is preferred that 5' terminal of one of the strands to be removed should be dephosphorized in advance. That is, a double-stranded DNA of interest carrying an extruded strand at only one end is dephosphorized at 5' terminal using phosphatase, and excised to obtain a desired fragment in which only one 5' terminal has been dephosphorized.

iv) The hydroxy group of 3' terminal of a double-stranded DNA is activated by introducing into the hydroxy group a trichlorotriazine group. On the other hand, the hydroxy group of 5' terminal is subjected to dephosphorization as described above, and trichlorotriazine is reacted with the resultant exposed hydroxy group for activation. Since reactivity of the hydroxy group at 5' terminal is much higher than that of the hydroxy group at 3' terminal, the former is exclusively activated. This method requires no extra nucleotide molecule on one strand of the double-stranded DNA.

The single-stranded DNA immobilized on a carrier, which is prepared according to the method of the present invention, is the one in which every DNA strand is immobilized on the carrier at its terminal. Accordingly, the immobilized DNA can behave substantially in the same manner as free DNA in term of its reactivity in hybridization reaction. Specifically, hybridization efficiency of the immobilized DNA of the invention is 2-10 fold higher than conventional immobilized DNAs. The conventional immobilized DNA cannot be used for the purpose of detecting a single nucleotide mutation in a test sample because the conventional immobilized DNA contains considerable amount of DNA strands which are immobilized on a carrier at a non-terminal nucleotide molecule, which destroys complementarity of said strands. However, the immobilized DNA of the invention allowed one to detect a mutation at a single nucleotide in a test sample. This means that the single-stranded DNA of the invention does not contain non-terminal nucleotide moelcule which has been bonded to the carrier.

Fig. 1 of the accompanying drawing is UV spectrum of the immobilized single-stranded DNA of the invention.

The following detailed examples are presented by way of illustration of certain specific embodiments of the invention.

## Example 1

The DNA fragments having the following sequences were prepared by the use of DNA synthesizer supplied by ABI at a 1μmole scale.

(1) 5' X GTC TGG GAA AAA CCC CCT TTG AGT 3'
(2) 5' ACT CAA AGG GGG TTT TTC CCA GAC 3'
Note: X means Amino Link 2 (ABI)

DNA franment (1) and DNA fragment (2) were obtained in an amount of 1.8mg and 1.3mg respectively, when they were purified according to the recommendation provided by the manufacturer of the DNA synthesizer. Purified solution containing 100μg of DNA fragment (1) was evaporated to dryness under reduced pressure. Purified solution containing 100μg of DNA fragment (2) was treated in the same manner. The fragment (1) (100μg) was dissolved in 1M NaCl (100μl) and combined with the fragment (2) (100μg). To the mixture was added 0.4M $NaHCO_3$ (pH7.5, 100μl). The resultant mixture was warmed on a water bath at 95°C for 2 minutes and then cooled to 55°C over 20 minutes. To the cooled mixture was added silica gel (20mg) activated by trifluoroethane sulfonyl chloride according to the method described in detail hereinafter, and the mixture was allowed to react at room temperature for 24 hours. After completion of the reaction, the silica gel was washed three times with 1M NaCl and then suspended in 1M NaCl (1ml). The suspension was warmed at 95°C for 5 minutes and centrifuged (12000 rpm) immediately. The resultant supernatant was discarded, and immobilized DNA fragment (1) was obtained as the residue. The yield of the immobilized DNA fragment molecules was determined using a spectrophotometer in the following manner.

A saturated sucrose solution was used as a solvent for dissolving the immobilized DNA fragment. After adjustment of the refractive index of the resultant solution, the absorbance of the solution was measured. This method minimizes an adverse influence of light scattering caused by gel and allows precise

determination.

As a spectrophotometer, Shimazu UV-200 was used. As a control, 5mg silica gel (shim pack diol)/400μl saturated sucrose solution was used. Fig. 1 shows the absorbance when 5mg immobilized fragment (1)-/400μl saturated sucrose solution was measured after background corrections using the control. Fig. 1 shows a specific absorbance at 260nm due to the DNA fragment.

The yield of the DNA fragment molecules which had been immobilized on silica gel was calculated in the following manner based on the fact that Absorbance 1.00 at 260nm corresponds to 30μg DNA fragment/ml.

$\Delta A_{260}$ reads 0.45 from Fig. 1, which corresponds to 13.5μg/ml (∵ 0.45×30). Control (5mg/400μl) equals to control (12.5mg/ml). Accordingly, calculation gives:
$13.5 \div 12.5 \times 1000 = 1.08$mg DNA/g silica gel.

Based on the above calculation, it revealed that 1.08mg of a single-stranded DNA was immobilized on 1g of silica gel.

Activation of silica gel by trifluoroethane sulfonyl chloride

The following manipulations should be conducted in a dry box or sterilized pack filled with nitrogen gas and with a caution to avoid a penetration of moisture.

1) Acetone and pyridine are dehydrated with molecular sieve.
2) The dehydrated acetone and pyridine, and a small stirrer chip are placed in a 10ml messflask.
3) Silica gel (1g) is quickly washed with acetone and then dehydrated acetone on a glass filter (#5 mesh) under suction with an aspirator, and placed in the above messflask.
4) The silica gel is vigorously stirred in the flask under a nitrogen atmosphere, and trifluoroethane sulfonyl chloride (Fluka) is dropwised added over one minute. During the above procedure, the flask is kept at about 0°C by ice-cooling.
5) After putting a stopper on the flask, the silica gel is allowed to react with the activating agent at about 0°C for 20 minutes with a decreased stirring speed so that the gel may not be broken.
6) After completion of the reaction, the gel is filtered on a glass filter and washed with acetone, acetone + 5mM HCl (1:1), and 5mM HCl.
7) The gel is washed again with acetone and dried on the filter by removing the acetone.
8) The gel is transferred to an egg-plant type flask, and residual acetone is removed under reduced pressure to give a silica gel activated by trifluoroethane sulfonyl chloride. This procedure should be conducted with ice-cooling because trifluoroethane sulfonyl chloride is unstable at elevated temperature.

Example 2

The DNA fragments having the following sequences were prepared by the use of DNA synthesizer (ABI) at a 1μM scale.

(1) 5' X GTC TGG GAA AAA CCC CCT TTG AGT 3'
(2) 5' ACT CAA AGG GGG TTT TTC CCA GAC 3'
Note: X means Amino Link 2 (ABI)

DNA fragments (1) and (2) were obtained in an amount of 1.2mg each, when they were purified according to the recommendation of the manufacturer of the DNA synthesizer.

Purified solution containing 500μg of the DNA fragment (1) was evaporated to dryness under reduced pressure, and the dried DNA fragment was dissolved in 1M NaCl (100μl). Purified solution containing 500μg of the DNA fragment (2) was evaporated to dryness, and the dried fragment was added to the resultant solution. The solution was warmed at 95°C for 2 minutes and then cooled to 35°C over 60 minutes. To the solution were added 0.4M $NaHCO_3$ (pH7.5, 100μl) and subsequently Tresyl 5PW gel (20mg, TOSOH), and the mixture was allowed to react at room temperature for 24 hours. After completion of the reaction, the Gel was washed three times with 1M NaCl and suspended in 0.5M NaCl (1ml). The suspension was warmed at 95°C for 5 minutes and centrifuged (12000 rpm) immediately. The supernatant was discarded and an immobilized DNA fragment was obtained as the residue. The amount of DNA fragment molecules immobilized on the gel was determined on the basis of the amount of uncaptured DNA fragment molecules

remained in the supernatant, which revealed 80μg immobilized DNA fragment/g dried gel ($A_{260}$ = 0.053, $A_{260}$ = 1 corresponds to 30μg DNA/ml).

## Claims

1. A method of immobilizing a single-stranded DNA on a carrier at the terminal, which comprises binding a double-stranded DNA comprising the single-stranded DNA and its complementary strand to the carrier only at the terminal of the single-stranded DNA and subsequently detaching the complementary strand from the single-stranded DNA by means of denaturing treatment, provided that the single-stranded DNA contains one or more extra nucleotide molecule(s) at the terminal when compared with the complementary strand or the terminal nucleotide molecule of the single-stranded DNA has been chemically modified so that the single-stranded DNA can bind to the carrier via the extra nucleotide molecule(s) or the chemically-modified terminal nucleotide molecule.

2. The method of Claim 1 wherein the carrier is a polymer.

3. The method of Claim 2 wherein the polymer is an organic polymer.

4. The method of Claim 3 wherein the organic polymer is the one into which a functional group has been introduced.

5. The method of Claim 2 wherein the polymer is an inorganic polymer.

6. The method of Claim 5 wherein the inorganic polymer is the one into which a functional group has been introduced.

7. The method of Claim 5 wherein the inorganic polymer is silica.

8. The method of Claim 6 wherein the inorganic polymer is silica.

9. The method of Claim 1 wherein the DNA is a synthetic DNA.

10. A single-stranded DNA immobilized at the terminal on a carrier, which is obtained by the method of Claim 1.

Fig. 1

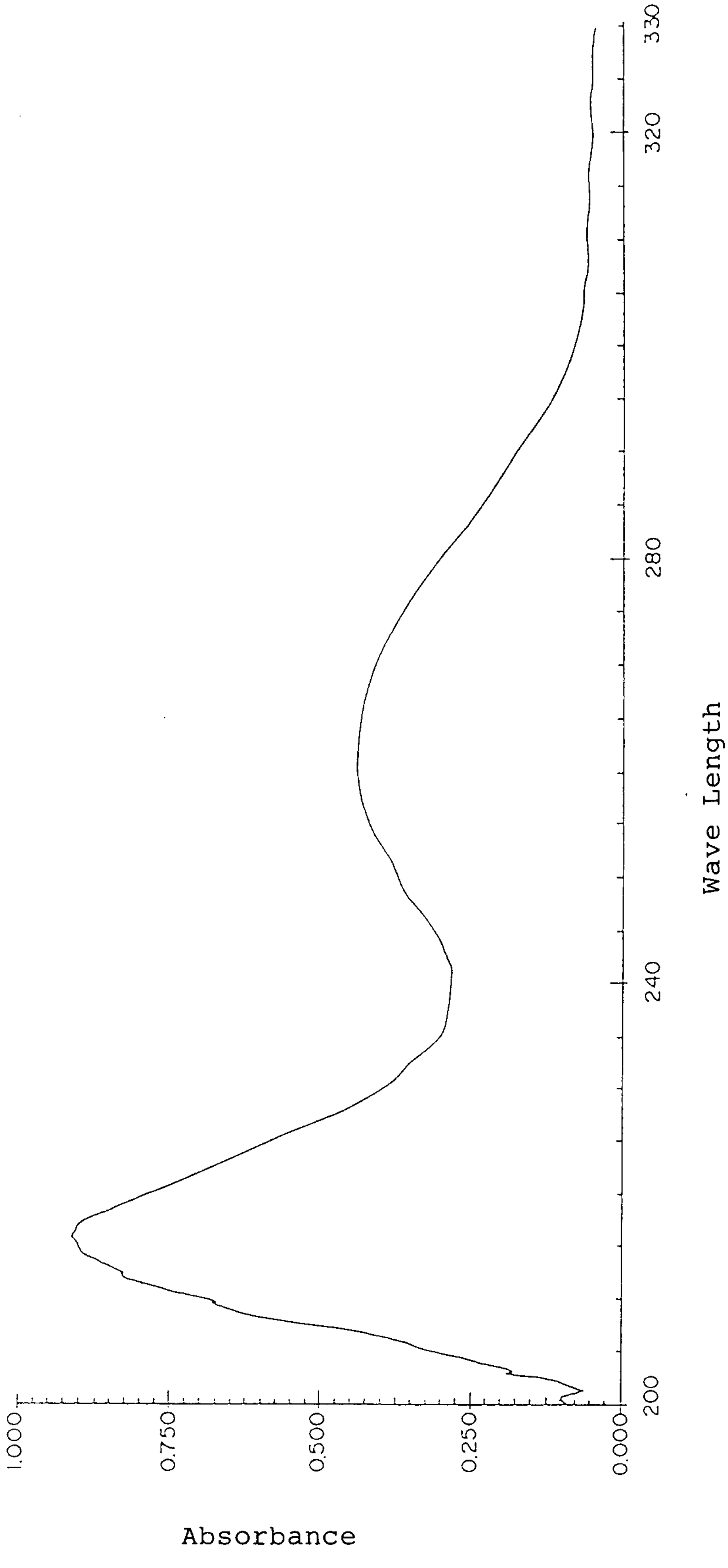

1.000
0.750
0.500
0.250
0.000
Absorbance
200
240
280
320
330
Wave Length

European Patent Office

# EUROPEAN SEARCH REPORT

Application Number

EP 91 11 9961

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5 ) |
|---|---|---|---|
| X | ANALYTICAL BIOCHEMISTRY<br>vol. 162, no. 1, April 1987, NEW YORK US<br>pages 130 - 136;<br>C. HANSEN ET AL.: 'Preparation of single-stranded deoxyribonucleic acid probes using an immobilized template'<br>* see the whole document, esp. : abstract; p.132, left column,l.30-right column, l.13; fig.1 *<br>--- | 10 | C12Q1/68 |
| X<br>A | WO-A-8 801 302 (SISKA DIAGNOSTICS,INC.)<br>* page 3, line 24 - page 5, line 34 *<br>* page 28, line 1 - page 34, line 36 *<br>--- | 10<br>1-9 | |
| Y | EP-A-0 172 153 (SMITHKLINE BECKMAN CORP.)<br>* page 3, line 10 - page 11, line 36; claims 1,3 *<br>--- | 1 | |
| Y | GB-A-2 197 720 (NATIONAL RESEARCH DEVELOPMENT CORP.)<br>* page 2, line 25 - page 3, line 21 *<br>* page 6, line 29 - line 34 *<br>--- | 1 | TECHNICAL FIELDS SEARCHED (Int. Cl.5 ) |
| A | WO-A-8 909 282 (CEMU BIOTEKNIK)<br>* page 3, line 1 - page 8, line 20; claims *<br>----- | 1,10 | C12Q |

The present search report has been drawn up for all claims

| Place of search | Date of completion of the search | Examiner |
|---|---|---|
| THE HAGUE | 16 MARCH 1992 | LUZZATTO E.R. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document